Europäisches Patentamt

**European Patent Office**

**Office européen des brevets**

(19)

(11) Publication number: **0 155 190**
A2

# (12) EUROPEAN PATENT APPLICATION

(21) Application number: **85301828.1**

(22) Date of filing: **15.03.85**

(51) Int. Cl.⁴: **C 12 N 15/00,** C 12 N 9/00, C 12 N 9/36

(30) Priority: **16.03.84 US 590102**
**11.09.84 US 649384**

(43) Date of publication of application: **18.09.85**
**Bulletin 85/38**

(84) Designated Contracting States: **AT BE CH DE FR GB IT LI LU NL SE**

(71) Applicant: **GENENTECH, INC., 460 Point San Bruno Boulevard, South San Francisco California 94080 (US)**

(72) Inventor: **Wetzel, Ronald Burnell, 455 Urbano Drive, San Francisco California 94127 (US)**

(74) Representative: **Armitage, Ian Michael et al, MEWBURN ELLIS & CO. 2/3 Cursitor Street, London EC4A 1BQ (GB)**

(54) **Non-naturally occurring zymogens and methods and materials for their construction.**

(57) Enzymes are converted from or into reversibly inactive forms, zymogens, by mutating one or more selected wild type amino acid residues, in the enzymes into residues that are capable of chemical inactivation and reactivation. The invention particularly is useful in expressing enzymes normally expected to be toxic to recombinant microbial hosts in the inactive or zymogen form, after which the zymogen is activated in order to produce the functional enzyme.

1

NON-NATURALLY OCCURRING ZYMOGENS AND METHODS

AND MATERIALS FOR THEIR CONSTRUCTION

The present invention relates to the field of protein engineering.

The publications and other materials used herein to illuminate the background of the invention and in particular cases to provide additional details respecting its practice are incorporated herein by reference and for convenience are numerically referenced in the following text and respectively grouped in the appended bibliography.

Proteins are one of a number of biologically functional chemical compounds. Proteins are unusual among biochemical compounds in the ability to catalyze reactions. Catalytic proteins are called enzymes.

All proteins are comprised of combinations of twenty building blocks called amino acids. The amino acids are linked together in linear unbranched fashion by a type of covalent bond called a peptide bond. The sequence of the amino acids so

2

linked is known as the primary structure of a protein.   A
sequence of amino acids is also often called a polypeptide.

The primary structure is important for at least
two reasons.   First, it is by far the most significant determinant of the three-dimensional conformation of a protein.
Second, the primary structure is the direct result of a linear
translation from the nucleotides of DNA.   Thus, essential
information regarding the sequence of DNA nucleotides can be
ascertained from the primary structure of a protein, and vice
versa.

The three-dimensional interactions of amino acids
determine the secondary and tertiary structure of a protein.
Secondary structure is the relationship or interaction between
neighboring amino acids of the primary chain.   It refers to a
regular, recurring arrangement in space of the polypeptide
chain along one dimension, e.g., a helical coil.

Tertiary structure is the relationship or interaction
between amino acids at some distance from each other in the
primary chain.   It refers to how the polypeptide chain is bent
or folded in three dimensions, to form the compact, tightly
folded structure of globular proteins.   Because of secondary
and tertiary structure, many complex interactions occur between
amino acids separated from each other within the primary sequence.   The result of these interactions is protein conformation.   This can be envisioned as a string of bar magnets, each
individual bar magnet representing an amino acid and the entire
string of magnets representing a protein.   If the string of
magnets is tossed in the air it will assume one of many possible
three-dimensional conformations.   However, the possibilities are

3

limited due to the physical properties of the magnets, i.e., because like magnetic poles repel, a three-dimensional conformation cannot exist with like poles next to each other.

Whatever the three-dimensional conformation, a change in the environment of a protein may alter the forces exerted on the protein with a comcomitant change in the conformation of the protein. For instance, the application of heat to a protein in solution often results in a conformational change. Further, if a high temperature is applied, a protein may not revert to its original conformation, even on cooling. The amino acid sequence of a polypeptide not only determines if and how it will achieve a stable, three-dimensional structure, but elements of the sequence also are critical to the activity of the polypeptide in other ways. For example, the chemical nature of portions of the folded polypeptide is critical to the interaction of that polypeptide with other molecules, such as the substrates of enzymes and the receptors of hormones.

Enzymes are proteins that catalyze biological reactions, those reactions occuring in the "active site" of the enzyme. Unlike other catalysts, which only increase reaction rate, enzymes and their active sites are specific for designated chemical reactants. Frequently, the reaction is so specific that one enzyme can react with only one chemical.

Two factors which determine this catalytic specificity are the three-dimensional folding of the enzyme and the amino acid sequence of the enzyme. Thus, a disruption or change in the three-dimensional conformation of an enzyme may cause a lessening, a substantial reduction, or a total loss of catalytic

4

activity. Such a change in catalytic activity via a change in protein folding is termed denaturation. Depending on the extent of the three-dimensional change or disruption the denaturation is reversible or irreversible.

Similarly, changes in the amino acid sequence of an enzyme may cause a lessening, a substantial reduction, or a total loss of catalytic activity, due to an alteration in the interaction of the folded enzyme with its substrate(s).

Similar conformational and chemical changes can influence the specific activity of other proteins, such as hormones. Conformational and chemical changes can be introduced into proteins in such a way that the new structural feature(s) can later be converted to the original protein. If in such a case, the new structural feature(s) causes a decrease in the activity of the protein, the introduction of the new structure(s) generates a zymogen.

Natural zymogens are widely found. These proteins exist in an inactive state in a cell or organism until they are changed into their active form, usually by the cleavage of one more specific peptide bonds that result in the removal of one (e.g. pepsinogen/pepsin) or more (e.g., chymotrypsinogen/chymotrypsin) short polypeptide sequences. The specific protein, be it hormone or enzyme, is usually denoted either by the prefix "pro", e.g., proinsulin, or by the suffix "ogen", e.g., trypsinogen. Proteins most commonly found in the zymogen form are those having strong signal (hormone) or catalytic (enzyme) functions. If these proteins remained in their active form even when not needed, they could cause extensive cellular or organic damage. Classical examples of naturally occurring

zymogens are the digestive enzymes -- those enzymes secreted into the gastrointestinal tract. These powerful digestive enzymes are secreted from cells of the gastrointestinal tract and activated only when needed to digest food, thus preventing them from digesting the gastrointestinal tract itself.

The only natural zymogens result from the structural modification of a protein through the addition of one or more polypeptide sequences which are cleaved to create the active form. Only when the active form of the protein is needed are these polypeptides cleaved to yield the active protein form. For instance, when the digestive enzyme trypsin is needed, the enzyme enterokinase is induced to cleave a sequence of six amino acids from trypsinogen, the inactive form of trypsin, to yield trypsin plus a hexapeptide. It is emphasized that this type of regulation is unidirectional for enzymes. No mechanisms are known that can transform enzymes back to their respective zymogens.

The present invention provides methods and means for constructing a non-naturally occurring zymogen by the change of one or more amino acids in a protein, such as a naturally occurring or wild-type protein. An embodiment is disclosed wherein a non-naturally occurring zymogen is created by altering one or more nucleotides of the DNA sequence coding for the naturally occurring or wild-type counterpart of the zymogen. Preferably, one or more acidic amino acid residues, aspartic acid and/or glutamic acid, are changed to the neutral amino acids asparagine and/or glutamine by any of several known methods for site-directed mutagenesis of DNA. The non-naturally occurring zymogen can be induced to return to its active form by appropriate chemical methods. Accordingly, the invention also provides methods and means for host cell production of zymogens, the active forms of which are normally toxic to that host cell. In a preferred embodiment the invention represents the first occassion in which a commercially significant bacteriacide has been microbially expressed as a zymogen, and shown to be easily converted to active form subsequent to expression. The invention also makes available, for the first time, a microbial source as the factory for the production of ample supplies of materials normally toxic to those microbes.

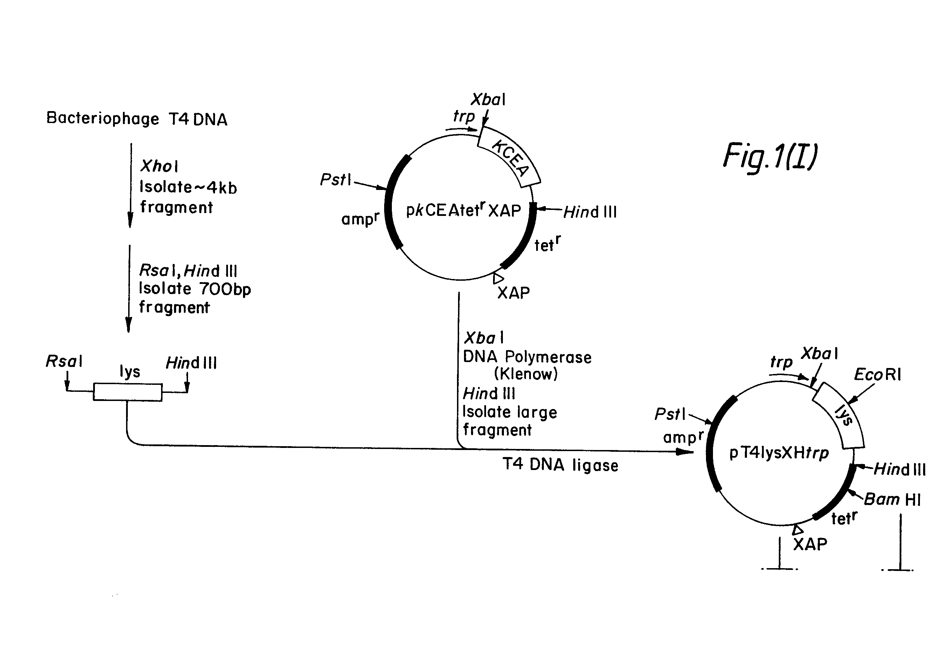

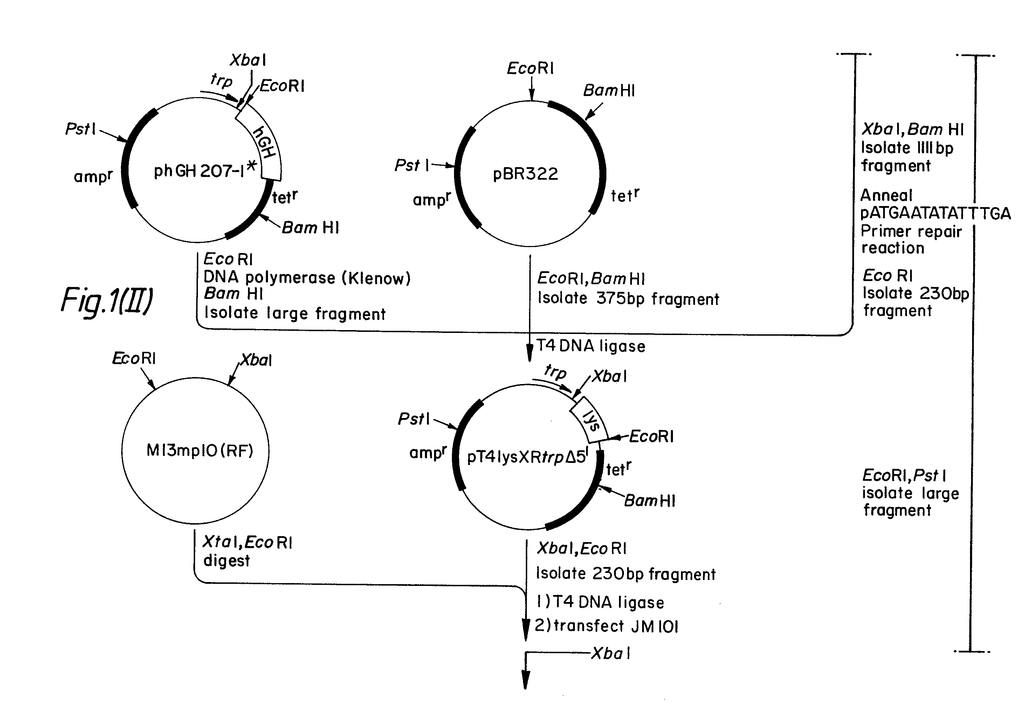

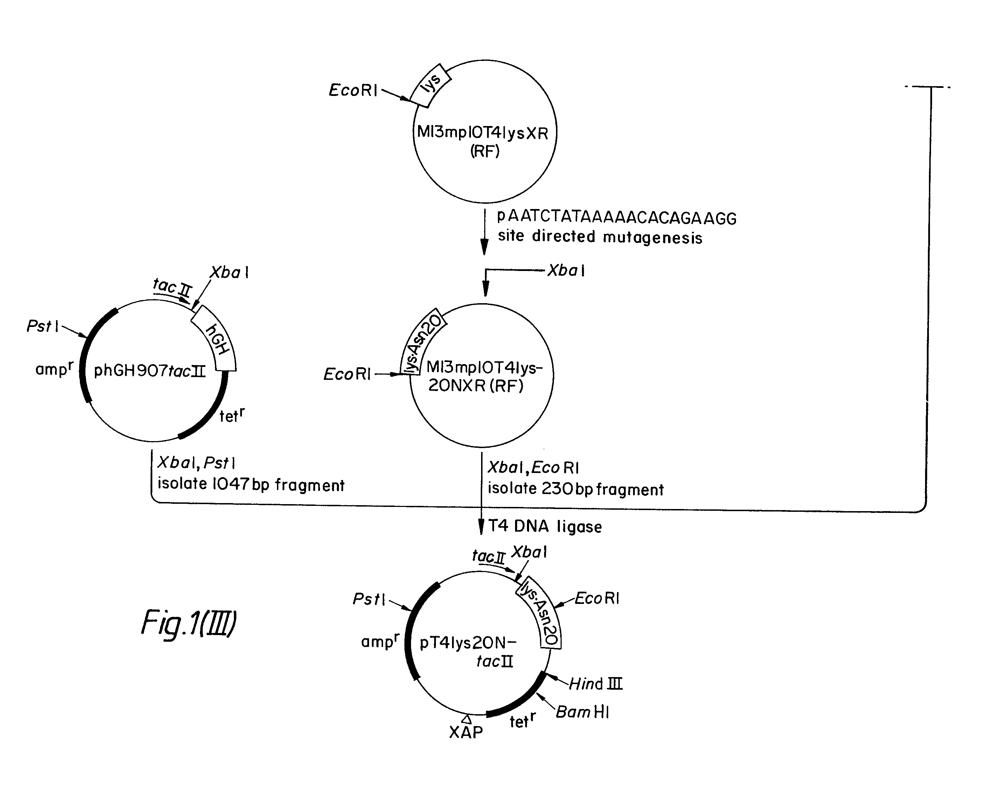

The manner in which these and other objects and advantages of the invention may be obtained will appear more fully from the detailed description which follows and from the accompanying drawing relating to a preferred embodiment of the invention, which depicts the synthetic scheme for construction of the lysozyme of the T4 bacteria virus containing an asparagine residue at position 20 in place of the naturally occurring aspartic acid residue.

With respect to the interpretation of this document, the following definitions apply:

1.    Replicable expression vector -- an extra chromo-somal length of duplex DNA comprising an intact replicon such that the vector can be replicated when placed within a host cell by transformation and will operate to express harbored DNA to produce a desired polypeptide.  A host cell so transformed is called a "transformant".  Presently, the replicable expression vectors commonly in use are derived from viruses and bacteria and most commonly are rings of bacterial DNA called "plasmids".

2.    Site-directed mutagenesis -- Any method of introducing a nucleotide change in a nucleotide sequence.  In one known method, a 15-base oligonucleotide is constructed so that it is complementary to a region of a DNA strand of known sequence, but with one to three mismatches.  When mixed with a clone of the complementary DNA strand, the oligonucleotide will anneal to it even though the match is not exact, as long as the hybridization conditions are not stringent and the mismatches are in the middle of the oligonucleotide segment. The segment then serves as a primer for the enzyme DNA polymerase I, which synthesizes the remainder of the complementary strand.  When the resulting double stranded molecule is introduced into E. coli, the molecule replicates to recreate either the original wild-type sequence or the mutant sequence.  The DNA containing the mutant sequence is isolated.

3.    Zymogen -- a polypeptide activatable via an induced structural change.

4.    Prolysozyme -- the zymogen of lysozyme.  No naturally occurring prolysozyme is known to exist.

8

5. Acidic residue -- any residue with a side chain ("R group") carboxyl moiety.

6. Wild-type -- a compound whose genetic or compositional construction is the same as the genetic or compositional structure of that compound as it most frequently occurs in nature.

7. Naturally occurring protein -- the amino acid sequence of a natural, non-altered, protein.

8. Active site -- that area of an enzyme in which the chemical alteration of a substrate occurs.

9. Residue -- any amino acid in a polypeptide.

10. Amido residue -- any residue with a side chain ("R group") amido moiety.

11. Carboxyl residue -- any residue with a side chain ("R group") carboxyl (COOH) moiety.

12. Critical carboxyl residue -- any carboxyl residue which may be substituted by a non-carboxyl residue to generate a zymogen.

13. Binding carboxyl residue -- any carboxyl residue which is a critical carboxyl residue by virtue of its role in the non-covalent interaction between an enzyme and its substrates and/or products.

14. Catalytic carboxyl residue -- any carboxyl residue which is a critical residue carboxyl by virtue of its chemical involvement in the mechanism of enzyme action.

15. Structural carboxyl residue -- any carboxyl residue which is a critical carboxyl residue by virtue of its role in maintaining an active conformation of a protein.

16. Nucleotide -- A monomeric unit of DNA or RNA consisting of a sugar moiety (pentose), a phosphate, and a nitrogenous heterocyclic base. The base is linked to the sugar moiety via the glycosidic carbon (1' carbon of the pentose) and that combination of base and sugar is a nucleoside. The base characterizes the nucleotide. The four DNA bases are adenine ("A"), guanine ("G"), cytosine ("C"), and thymine ("T"). The four RNA bases are A, G, C and uracil ("U").

17. Oligonucleotide -- any small group of nucleotides connected one to the other by phosphodiester bonds between the 3' and 5' carbons of adjacent pentoses.

18. DNA sequence -- any number of nucleotides connected one to the other by phosphodiester bonds between the 3' and 5' carbons of adjacent pentoses.

19. Codon -- A DNA sequence of three nucleotides (a triplet) which encodes through messenger RNA ("mRNA") an amino acid, a translational start signal or a translational termination signal.

20. Host -- Cells which on transformation by a replicable expression vector enable the expression vector to replicate and to accomplish its other biological functions, e.g., the production of protein through the expression of the DNA of the replicable expression vector.

The invention relates to the alteration of one or more wild type protein residues to other residues to create a zymogen. In a preferred form, the invention provides the construction of a zymogen, containing an amido residue at the position normally occupied by a critical carboxyl residue, which

10

may be converted to a protein containing the critical carboxyl residue by a process called deamidation. A preferred embodiment is disclosed wherein the aspartic acid residue at position 20 of the lysozyme of the bacteria virus T4 is altered to asparagine to create the zymogen prolysozyme.

The amido moieties on the side chains of glutamine and asparagine residues in polypeptides are susceptible to a process called deamidation in which hydrolysis of the amido moiety generates a molecule of ammonia and a residue whose side chain contains a carboxyl group. That is, the hydrolysis generates glutamic acid and aspartic acid residues. Such deamidation occurs under a variety of pH and temperature conditions. Under any given conditions, the rate at which a glutamine or asparagine residue deamidates will depend upon the amino acids which flank it in the primary sequence of the polypeptide chain. Robinson, A. B. and Rudd, C. J., Deamidation of Glutaminyl and Asparaginyl Residues in Peptides and Proteins, In: Curr. Top. Cell. Regul., Vol 8, pages 254-260 (1974).

Generally, the deamidation of residues in proteins and polypeptides has been considered a nuisance, because at least some asparagines and glutamines deamidate under such mild conditions that it is difficult to preserve those residues while handling or studying the polypeptide. For example, the deamidation of an asparagine residue during experiments to determine the amino acid sequence of a protein will lead to the mistaken assumption that there exists in the naturally occurring protein an aspartic acid residue at the position normally occupied by that asparagine. Ibid. at 253. Furthermore, during

0155190

11

the chemical synthesis of polypeptides, deamidation can occur during a number of steps, leading to an incorrect final synthetic product. Ibid. at 254. Deamidation of glutamines and asparagines during amino acid compositional analysis of polypeptides also renders it impossible to measure the percentage of these two amino acids by standard methods. Ibid. at 251. It has also been speculated that in many cases investigators may carry out experiments on proteins unaware that their structures vary from an assumed structure because of deamidation before or during the experiment. Ibid. at 253. Nevertheless, deamidation has been shown to be a desirable feature in one embodiment of this invention.

One method for identifying appropriate sites for zymogen formation begins by reviewing literature x-ray crystal structure data, amino acid or DNA sequence data, or other structure function data which may indicate whether a residue, for example, aspartic acid or glutamic acid, is critical to the function of the molecule by virtue of it containing in its side chain a negatively charged carboxyl group. If no such literature exists, such data may be established by independent investigation.

If none of the above structure function data allows for identification of a critical residue, an x-ray crystallograph may be examined, if available, to see if it might yield other information. For example, the x-ray crystallograph might aid in location of the active site, and the identification of, for example, one or more aspartic acid or glutamic acid residues which might be imagined to be critical

12

to the function of the molecule by virtue of their negatively charged carboxyl moieties.

If the above methods are not successful one may examine the amino acid sequence and "rank", for example, the aspartic acid residues, and separately the glutamic acid residues, by the hydrophilicity of the amino acids adjoining them, from highest to lowest hydrophilicity. Because neighboring amino acids with the highest degree of hydrophilicity provide an environment most suited for deamidation, aspartic acid residues and glutamic acid residues in those areas are good candidates for alteration.

After selection, the residue most likely to be required for activity, or in the most hydrophilic environment, is replaced by its amido R-group analog and the activity of this derivative is measured. It is to be noted that if the implicated residue is glutamic acid, and if its substitution by a glutamine is deactivating, it is also advisable to ascertain whether aspartic acid is tolerated, i.e., whether it may be substituted at that site without significant loss of activity. If so, the original glutamic acid may be replaced by asparagine and the activity of this derivative measured. In other words, notwithstanding that the wild type residue to be replaced is glutamic acid, it is advisable to determine whether it can replaced by glutamine and/or asparagine, as the rates of deamidation of glutamine and asparagine differ, and one deamidation rate may be preferred over another.

13

If the replacement of the selected amino acid does not effectively reduce activity, the next best choice should be tested, the next best, and so on. When a replacement is found which effectively reduces activity, the deactivated protein should be tested for its ability to acquire the activity of the wild type protein under various conditions catalyzing deamidation.

The generation of a zymogen by these methods, however, requires that the amido analog of the critical carboxyl residue deamidate under conditions which do not also deactivate the wild type protein (for example, through secondary deamidations of critical carboxamides, or other reactions). If the prospective zymogen does not activate as expected, the rate of deactivation of the wild type protein must be measured and compared to the rate of deamidation predicted for an amide such as that introduced to form the prospective zymogen. If the rates are comparable, the structural feature(s) which render the wild type protein sensitive to these conditions (e.g., a conformation which will denature, a sulfur atom which can oxidize) must be defined. After that determination, the alteration of the implicated feature(s) by protein engineering methods should enable a decrease in the deactivation rate of the wild type enzyme. This change should then be made in the prospective zymogen mutant and the zymogen mutant retested for the ability to activate.

Although it provides a method of general applicability for the production of useful activable zymogens, including proenzymes, the invention is particularly suited to

14

the expression of substances which in their wild type form are toxic or lethal. A preferred embodiment illustrative of the invention is next discussed, in which a gene coding for T4 prolysozyme is constructed, cloned, microbially expressed, and the prolysozyme recovered and activated.

Construction of pT4lysXHtrp

The accompanying drawing illustrates the construction of the replicable expression vector pT4lys20NtacII. A sample of cytosine containing T4 DNA (1) was digested with the restriction endonuclease XhoI and a fragment of about 4,000 base pairs was isolated by agarose gel electrophoresis. The purified fragment was then cleaved with the restriction endonucleases RsaI and HindIII to yield a gel-purified fragment of about 700 base pairs which contained the T4 lysozyme gene. The plasmid pKCEAtet XAP (2) was cleaved with the restriction endonuclease XbaI, filled in with DNA polymerase I (Klenow fragment) and subsequently digested with the restriction endonuclease HindIII. The gel purified vector fragment was ligated with T4 DNA ligase to the 700 bp fragment to yield the plasmid pT4lysXHtrp.

Construction of pT4lysXRtrpΔ5'

The plasmid phGH207-1*(3) was cleaved with the restriction endonuclease EcoRI and elongated with DNA polymerase I (Klenow fragment), then cleaved with the restriction endonuclease BamHI, and the large vector fragment was purified by gel electrophoresis. The plasmid pT4lysXHtrp was cleaved with the restriction endonucleases XbaI and BamHI to yield a

15

1111 base pair fragment. A 14-base oligonucleotide primer was used in a primer repair reaction to delete the 97 bases of untranslated T4 DNA on the 5' end of the lysozyme gene (4). About 0.5 micrograms of the primer was labeled using $^{32}$P-ATP and polynucleotide kinase. The gel purified XbaI-BamHI fragment of pT4lysXHtrp was annealed to the labeled primer by heating to 95-100C for 4 minutes followed by quick freezing. Nucleotide triphosphates were then added to the frozen mixture, which was then allowed to thaw. When the mix was partially thawed, DNA polymerase I (Klenow fragment) was added. The reaction was incubated at 37C for 1.5 hours. EDTA was added to stop the reaction. The DNA was washed with phenol and chloroform, precipitated and washed with ethanol. The fragment was identified on an acrylamide gel by autoradiography. The identified fragment was eluted from the gel and quantified by scintillation counting. The resulting fragment was then digested with the restriction endonuclease EcoRI to yield a fragment of 230 base pairs which was isolated by gel electrophoresis. The plasmid pBR322 was digested with the restriction endonucleases EcoRI and BamHI and the 375 base pair fragment purified by gel electrophoresis. A three-way ligation reaction was performed with the three fragments to construct the pT4lysXRtrp$^{\Delta}$5' plasmid.

This ligation mixture was used to transform E. coli K-12 294 (ATCC No. 31446). Plasmid DNA was isolated from the resulting transformants and applied to nitrocellulose paper. The paper was treated with $^{32}$P labeled primer. When the nitrocellulose was gradually washed under conditions of increasing hybridization stringency, 37 positive sequences were seen. Six of the most strongly hybridizing positives were picked for M13 sequencing.

The plasmid isolated from each of these strongly hybridizing clones was digested with the restriction endonucleases EcoRI and XbaI and the 230 base pair fragment was gel purified. The double stranded RF form of the M13mp10 DNA was also digested with EcoRI and XbaI. The 230 base pair fragment was ligated into the digested M13 vector fragment overnight at room temperature. The ligation reaction mixtures were used to transfect E. coli JM101 (ATCC No. 33876), and plaques were picked and sequenced by standard methods. Only one of the positives analyzed by DNA sequencing had the correct sequence. The replicable expression vehicle which contains the EcoRI/XbaI fragment of the correct sequence was designated pT4lysXRtrp 5'.

### Construction of pT4lys20NtacII

The method of site-directed mutagenesis was used to construct a zymogen gene with an asparagine codon replacing an aspartic acid codon at the site of the T4 lysozyme gene corresponding to the position 20 amino acid of T4 lysozyme. A 230 base pair fragment of the replicable expression vector pT4lysXRtrpΔ 5' containing the 5' half of the lysozyme gene was isolated after treatment of the replicable expression vector with the restriction endonucleases XbaI and EcoRI and was cloned into the phage M13mp10(2). The synthetic oligomer pAATCTATAAAAACACAGAAGG was used to mutate the aspartic acid codon GAC, located 58 base

17

pairs away from the lysozyme gene start codon ATG, to the asparagine codon AAC. After confirmation by M13 DNA sequencing that the desired mutation had occurred (6), the mutated DNA ·fragment was cleaved out of the RF form of M13mp10 with the restriction endonucleases XbaI and EcoRI. This fragment was ligated to the 1047 base pair fragment cleaved from the replicable cloning vehicle phGH907tacII with the restriction endonucleases XbaI and PstI, and to the large fragment of the replicable expression vector pT4LysXHtrp which had been cleaved with EcoRI and PstI. This litigation mixture was used to transform E. coli D1210 (ATCC No. 31449). The transformants were grown and selected on LB/ampicillin plates. Replicable expression vectors (REVs) were isolated from cells grown from the colonies and analyzed by restriction mapping. Colonies containing REVs with restriction fragments of the expected size, consistent with the desired final construction, were again grown. A SDS polyacrylamide gel with crude cellular protein from each IPTG induced culture indicated the presence or absence of an induced protein of about 19,000 molecular weight. The REV isolated from a culture which produced this new protein but which did not lyse after a freeze thaw cycle was designated pT4lys20NtacII.

Extraction and Purification of T4 Lysozyme (Asp 20 → Asn)

A colony of E. coli D1210/pT4lys20NtacII was picked from a culture plate (LB broth/ampicillin) from a fresh transformation and grown overnight at 37 in five ml of LB broth containing 20 micrograms/L ampicillin. This culture was

18

used to inoculate 1 liter of M9 medium containing 20 micrograms/ ml ampicillin in a 4 L shake flask. When the culture reached an OD550 of about 2, or late log phase growth, it was induced with the addition of 1mM isopropyl-beta-D-thiogalactoside. After one hour the cells were harvested by centrifugation and frozen.

Frozen cells from 0.5 L of culture were lysed by sonication at 0 in 20 ml 50mM tris HCl, pH 7.5, 1mM EDTA, 1mM phenylmethylsulfonyl fluoride. Two milliliters of five percent polyethylene imine hydrochloride (pH 7.5) was added with stirring and the suspension centrifuged at 10,000 rpm for 20 minutes in a Sorvall SS-34 rotor. The supernatant was passed over a DEAE-cellulose column and washed with 50mM tris HCl, pH 7.5, 1mM EDTA.

The flow through fractions containing 0.3 OD280 or higher were pooled and adjusted to pH 5.2 with concentrated acetic acid. This was loaded onto a CM-cellulose column (10ml bed volume in a 1 cm diameter column at 4) equilibrated with 50mM NaOAc, pH 5.5, 1mM EDTA, 1mM 2-mercaptoethanol. The column was washed with equilibration buffer, then eluted with a linear gradient of 0-0.5m NaCl in equilibration buffer, total volume 100 ml, at a flow rate of 10ml/hr. The predominant OD280 peak at a [NaCl] of about 0.25 M contained a protein shown by SDS polyacrylamide gel electrophoresis to be of the right molecular weight. The yield of protein was 3 milligrams. This material displayed a slight amount of lysozyme activity, 0.3 units/microgram, corresponding to 0.02% of the specific activity of the wild type enzyme.

Activation of T4 lysozyme (Asp 20 → Asn)

T4 lysozyme (Asp 20 ──→ Asn) was heated at 48 at a concentration of 32 micrograms/ml in 100mM potassium phosphate, pH 9, 0.1M NaCl, 7.2 M urea. After 10-30 minutes, the reaction mixture exhibited a specific activity of 15 units/microgram, representing an activation of 50-fold over the material that was isolated from E. coli. In another process, T4 prolysozyme (Asp 20 ──→ Asn) was heated at 48°C at a concentration of 32 micrograms/ml in 100mM potassium phosphate, pH 9, 0.1M NaCl. After 10-30 minutes, the reaction mixture exhibited a specific activity of 2.3 units/microgram, representing an activation of about 8-fold over the material isolated from E. coli.

It is understood that various other modifications will be apparent to and can readily be made by those skilled in the art without departing from the scope and spirit of this invention. Accordingly, it is not intended that the scope of the claims appended hereto be limited to the description as set forth herein, but rather that the claims be construed as encompassing all the features of patentable novelty which reside in the present invention, including all features which would be treated as equivalents thereof, by those skilled in the art to which this invention pertains.

BIBLIOGRAPHY

1. O'Farrell et al., Molec. Gen. Genet. 179:421-435 (1980); Owen et al., J. Mol. Biol. 165:229-248 (1983).

0155190

20

2. Cabilly et al., <u>Generation of Antibody Activity from Immunoglobulin Polypeptide Chains Produced in Escherichia coli.</u>, Proc. Nat'l. Acad. Sci. USA (1984), <u>81</u>, 3273.

3. Gray, et al., <u>Bio/Technology</u>, <u>2</u>, 161-165.

4. DeBoer et al., In: <u>From Gene to Protein: Translation into Biotechnology; Miami Winter Symposia</u>, Ahmad et al. (eds), Academic Press, New York, <u>19</u>, 309-327 (1982).

5. Goeddel et al., <u>Nucleic Acids Res.</u> 8:4057-4074 (1980).

6. Zoller, M.J. and Smith, M. (1983) In: <u>Methods in Enzymology</u>, Wu, R. et al. (eds.) Academic Press, New York, <u>100</u>, 468-500.

7. Messing, J. (1983) In: <u>Methods in Enzymology</u>, Wu, R. et al. (eds.) Academic Press, New York, <u>101</u>, 20-78.

CLAIMS

1.    A non-naturally occurring zymogen.

2.    The zymogen of claim 1 wherein at least one acidic residue of a wild-type enzyme is changed to a neutral residue.

3.    The zymogen of claim 2 wherein the R group of the acidic residue contains a carboxyl group.

4.    The zymogen of claim 2 or claim 3 wherein the R group of the neutral residue contains an amido group.

5.    The zymogen of any one of claims 2, 3 and 4 wherein the acidic residue is a critical residue, a catalytic residue, a structural residue or a binding residue.

6.    The zymogen of any one of claims 2 to 5 wherein the wild-type enzyme is T4 lysozyme and the acidic residue is the aspartic acid found at position 20 of said T4 lysozyme.

7.    The zymogen of claim 6 wherein the position 20 residue of said T4 lysozyme is changed from aspartic acid to asparagine.

8.    The zymogen of claim 2 wherein the ratio of aspartic acid changed to asparagine is about 1:1 and/or the ratio of glutamic acid changed to glutamine is about 1:1.

9. DNA coding for a zymogen of any one of the preceding claims.

10. A replicable expression vector which includes the DNA of claim 9.

11. The replicable expression vector pT4Lys20NtacII.

12. A host cell which has been transformed with the replicable expression vector of claim 10 or claim 11.

13. A process for producing a zymogen which comprises culturing transformed host cells of claim 12.

14. A method for constructing a non-naturally occurring zymogen comprising the alteration of at least one residue of its counterpart protein.

CLAIMS

1.   A method for constructing a non-naturally occurring zymogen comprising the alteration of at least one residue of its counterpart protein.

2.   The method of claim 1 wherein at least one acidic residue of a wild-type enzyme is changed to a neutral residue.

3.   The method of claim 2 wherein the R group of the acidic residue contains a carboxyl group.

4.   The method of claim 2 or claim 3 wherein the R group of the neutral residue contains an amido group.

5.   The method of any one of claims 2, 3 and 4 wherein the acidic residue is a critical residue, a catalytic residue, a structural residue or a binding residue.

6.   The method of any one of claims 2 to 5 wherein the wild-type enzyme is T4 lysozyme and the acidic residue is the aspartic acid found at position 20 of said T4 lysozyme.

7.   The method of claim 6 wherein the wild-type enzyme is T4 lysozyme and the position 20 residue of said T4 lysozyme is changed from aspartic acid to asparagine.

8. The method of claim 2 wherein the ratio of aspartic acid changed to asparagine is about 1:1 and/or the ratio of glutamic acid changed to glutamine is about 1:1.

9. A process for producing a non-naturally occurring zymogen which comprises culturing cells transformed with a replicable expression vector which includes DNA coding for the zymogen, the coding sequence specifying an alteration of at least one residue of the counterpart protein to the zymogen as defined in any one of the preceding claims.

10. A process according to claim 9 wherein the replicable expression vector is pT4Lys20NtacII.

Fig.1(I)

Fig.1(II)

Xbal
trp
EcoRI

Pst I
ampr
phGH 207-I*
hGH
tetr
Bam HI

EcoRI
DNA polymerase (Klenow)
Bam HI
Isolate large fragment

EcoRI
BamHI

Pst I
ampr
pBR322
tetr

EcoRI, Bam HI
Isolate 375bp fragment

Xbal, Bam HI
Isolate IIIIbp
fragment

Anneal
pATGAATATATTTGA
Primer repair
reaction

Eco RI
Isolate 230bp
fragment

T4 DNA ligase

EcoRI
Xbal

MI3mplO(RF)

Xtal, EcoRI
digest

trp
Xbal

Pst I
ampr
pT4lysXRtrpΔ5'
lys
EcoRI
tetr
BamHI

Xbal, EcoRI

Isolate 230bp fragment

1) T4 DNA ligase
2) transfect JM IOI

Xbal

EcoRI, Pst I
isolate large
fragment

2/3

0155190

Fig.1(Ⅲ)